# EUROPEAN PATENT SPECIFICATION

(11) **EP 0 927 003 B1**
(45) Date of publication and mention of the grant of the patent: **24.04.2002**
(21) Application number: 97936417.1
(22) Date of filing: 08.08.1997
(51) Int. Cl.: A61C 15/04, A61K 7/16, D01F 8/06, D01F 8/12, D01F 8/14

(54) **DENTAL FLOSS**
ZAHNSEIDE
FIL DENTAIRE

(30) Priority: 15.08.1996 US 699891
(43) Date of publication of application: 07.07.1999
(73) Proprietor: GILLETTE CANADA COMPANY, Mississauga, Ontario L4Z 4C5 (CA)
(72) Inventor: TSAO, Belinda, Los Altos, CA 94022 (US); PARK, Edward, Hosung, Sharon, MA 02067 (US); ZWICK, Paul, Stow, OH 44224 (US); DESAI, Pranav, Anaheim Hills, CA 92807 (US); TSENG, Mingchih, Michael, Hingham, MA 02043 (US); CHIANG, Casper, W., Danville, CA 94526 (US)
(74) Representative: Ebner von Eschenbach, Jennifer
(86) International application number: PCT/US97/13802
(87) International publication number: WO 98/06350

(56) References cited:
- EP-A- 0 686 390
- WO-A-93/02633
- WO-A-96/39117
- US-A- 4 583 564
- US-A- 4 798 216
- US-A- 5 262 468

## Description

This invention relates generally to the field of filaments and extrusion methodology for producing such and more particularly relates to multicomponent floss materials and methods for producing such.

Tooth decay and dental disease can be caused by bacterial action resulting from plaque formation around the teeth and/or the entrapment of food particles in interstices between teeth. Removal of plaque and entrapped food particles reduces the incidence of caries, gingivitis, and mouth odors as well as generally improving oral hygiene. Conventional brushing has been found to be inadequate for removing all entrapped food particles and plaque. To supplement brushing, dental flosses and tapes are used.

Monofilament, single component elastomeric fibers have been produced from various materials. When the material is soft enough for use as a floss, however, these monofilaments sometimes are not strong enough to withstand forces experienced during flossing, and are susceptible to shredding or tearing. An improved thermoplastic elastomer gelatinous composition with high elongation and tensile strength properties and suitable for use as dental floss is disclosed in U.S. 5,262,468.

Dental flosses often include additives such as flavors or colors. These flavors have been conventionally applied by coating the additive onto the surface of the floss. Some dental flosses and tapes have been coated with waxes to aid in insertion of the floss or tape between the teeth.

Improved dental flosses are formed of a single filament that includes two or more coextruded thermoplastic elastomer ("TPE") components selected to provide desired properties to the floss. Manufacturing floss from two or more different TPE components can provide the floss with the desired characteristics of each component (e.g., good tensile strength, slipperiness, and comfort during use), which otherwise might be unavailable from a single component. For example, in a core-and-sheath arrangement, the inner core TPE may provide greater tensile strength, viscoelasticity, resiliency and tear-resistance to the fiber than are available with the sheath material alone; the outer sheath material may provide a desired surface property, such as slipperiness or softness or abrasiveness, to improve ease of insertion, comfort/"mouth feel" and cleaning capability, respectively, of the floss; either material may serve as a carrier for additives, such as flavors, scents and medicaments. U.S. 4,583,564 discloses a dental floss consisting of higher melting point core material and lower melting point sheath material allowing easier insertion between the teeth.

In a sheath-and-core arrangement, TPE components are selected to ensure that the sheath and core are sufficiently adhered to each other so that separation during use is avoided. The monofilament's properties can be adjusted through properly blending different TPEs while retaining proper adhesion between the core and sheath. Adhesion may also be improved by varying the surface area of the core, or increasing the number of filaments comprising the core.

When relaxed, the diameter of multicomponent TPE monofilament floss of the invention may be too large to insert between teeth for flossing. Due to the viscoelasticity of the TPE materials, however, the floss is capable of being stretched before use, thereby decreasing the diameter of the monofilament for easier insertion between the user's teeth.

In accordance with the invention, there is provided a multicomponent, monofilament, viscoelastic dental floss characterized by a core composed of a first thermoplastic elastometer and a sheath encasing said core composed of a second, different thermoplastic elastometer, the difference of the melting points of said first and second thermoplastic elastomers being less than 30°C., said floss having a Shore A hardness of less than 10 and an ultimate elongation of greater than 300, said floss having a tear resistance of greater than 40 cycles on a blunt-toothed fray test.

In a preferred embodiment, the dental floss is characterized by a plurality of continuous cores having a first thermoplastic elastomer and a body sheath substantially adhered to and substantially surrounding each of said cores along substantially the entire lengths thereof comprising a second thermoplastic elastomer, the difference of the melting points of said first and second thermoplastic elastomers being less than 10°C. (50°F.), said floss having a Shore A hardness of less than 10 and an ultimate elongation of greater than 300, said floss having a tear resistance of greater than 40 cycles on a blunt-toothed fray test. The plurality of continuous cores can be arranged in multiple groupings of cores spaced throughout the floss' cross-sectional area.

A further aspect of the invention features improved multicomponent dental flosses in which one or more of the components includes an additive, e.g., a color, fragrance, flavor or active ingredient, which is releasable from the floss.

The improved flosses of the invention can be made by a method which includes (a) coextruding two or more TPE polymers through a multicomponent die to form a multicomponent filament of a specific geometry; and (b) treating the filament for use as a dental floss by, for example, creating a textured surface.

Alternatively, TPE gel can be applied to floss by pressure-coating or "pulltruding" the core filament(s) of one or more components, which can then also be textured.

Other features and advantages of the invention will be apparent from the drawings, the following Detailed Description:
Fig. 1 is a schematic view of a horizontal production line for manufacturing a coextruded dental floss according to one embodiment of the invention.

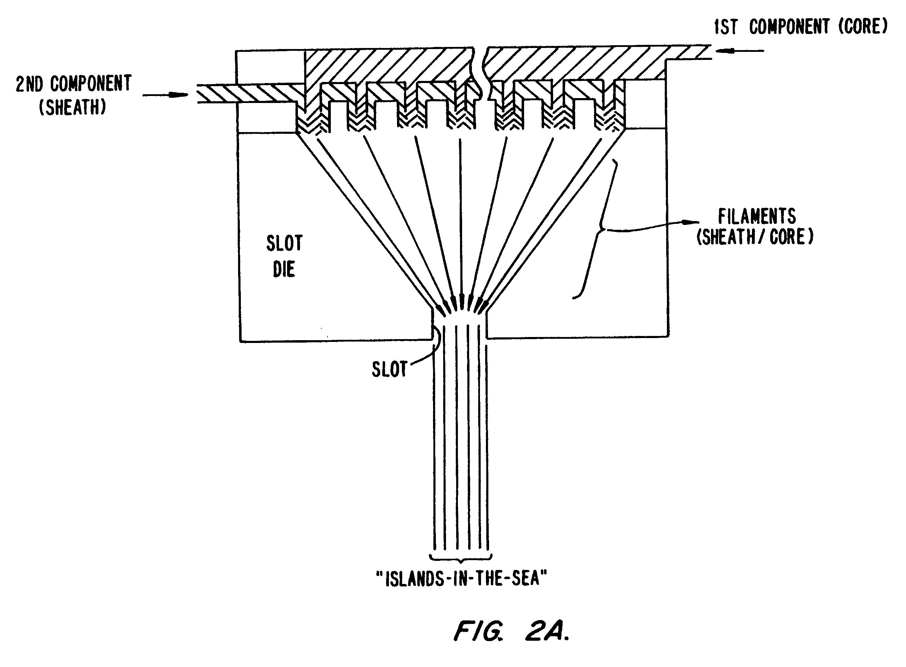

Fig. 2 is a cross-sectional view of a spinneret usable to produce multicomponent, coextruded flosses of the present invention.
Fig. 2a shows the die-and-slot arrangement for producing the "islands-in-the-sea" tape embodiment.
Figs. 3a - 3d are cross-sectional views, taken radially, of multicomponent coextruded filaments having cores with various cross-sections to increase the degree of adhesion between the core and sheath materials, either by increasing the surface area alone (Figs. 3a, 3e and 3f) or by also using mechanical interlocking structures (Figs. 3b and 3d).
Figs. 4 - 4f are cross-sectional views, taken radially, of a trilobal single component filament according to one embodiment of the invention, a trilobal multi-component filament having a sheath/core cross-section, a trilobal multi-component filament having a tipped cross-section, and filaments having four and six square bumps and four and six triangular bumps to create a textured surface, respectively.
Figs. 5a - 5d are cross-sectional views of various embodiments of a floss having core filaments embedded in a gel body: an 11 x 12 x 11 tape; an 11 x 12 x 11 rod; a rod floss having five distinct groups of eight core filaments each; and a floss having 39 filaments arranged in three concentric circles surrounding a single core filament all embedded in gel.

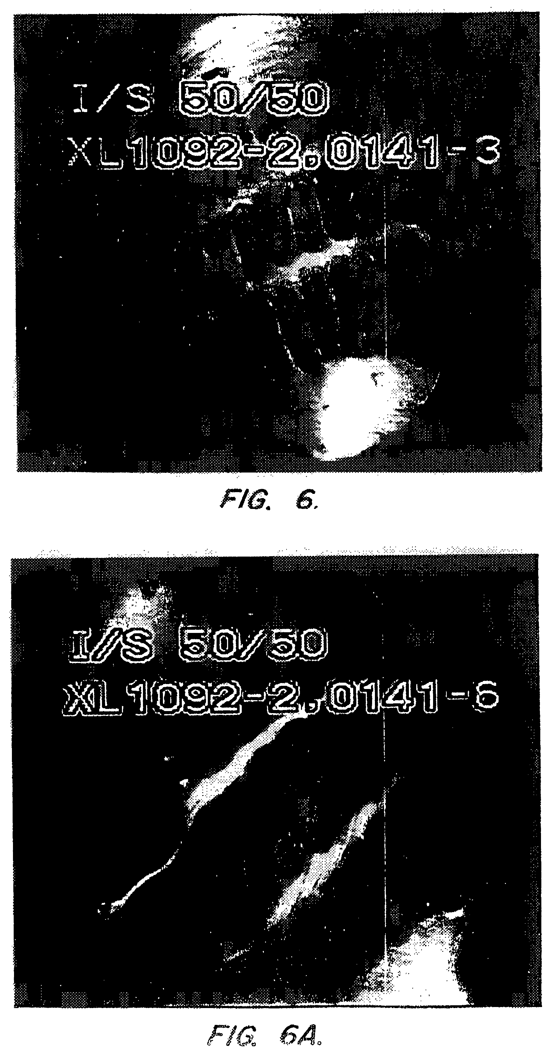

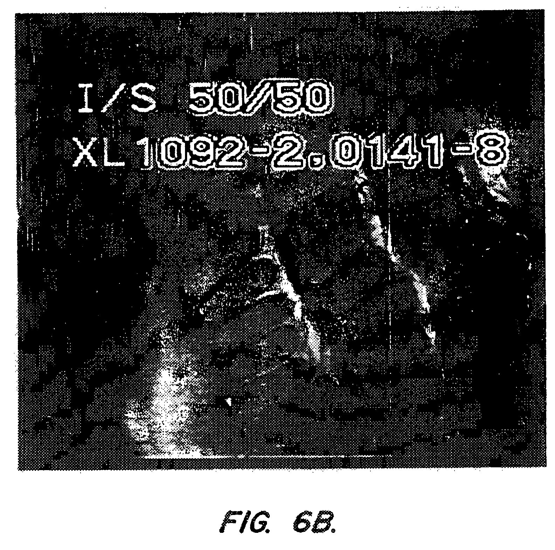

Figs. 6 - 6b are optical micrographs of "islands-in-the-sea" dental floss of the invention at 300X magnification.
Fig. 7 depicts a spiral-textured floss.
Fig. 8 depicts the equipment set-up used to conduct the fray test.

Before the present monofilament, multicomponent dental flosses and processes for extruding such are described, it is to be understood that the invention is not limited to the particular embodiments or extrusion methodologies described. Such floss components and methodologies may, of course, vary. It is also to be understood that the terminology used herein is for the purpose of describing particular embodiments only, and is not intended to be limiting. Instead, the scope of the present invention will be established only by the appended claims.

It must be noted that as used in this specification and the appended claims, the singular forms "a", "an" and "the" include plural reference unless the context clearly dictates otherwise. Thus, for example, reference to "a polymer" includes mixtures of different polymers.

Unless defined otherwise all technical and scientific terms used herein have the same meaning as commonly understood to one of ordinary skill in the art to which this invention belongs. Although any methods and materials similar or equivalent to those described herein can be used in the practice or testing of the present invention, the preferred methods and materials are now described. All publications mentioned herein are incorporated herein by reference.

### Definitions

By "multicomponent", we mean that the filaments have two or more components, each comprised of a different thermoplastic material; by "coextruded", we mean that at least two of the components are present in the form of substantially separate phases having a distinct interface between them, rather than being intermixed after simultaneous but separate extrusion. The filaments are preferably formed by processes which are referred to in the art as "coextrusion", but the term "multi-component coextruded", as used herein, encompasses filaments having the structure described herein which are manufactured by processes other than coextrusion. The term "dental floss", as used herein, is defined to include dental flosses, dental tapes, and similar articles, that are sized to be drawn between the teeth.

The dental flosses of the present invention include an inner core and an outer sheath of a soft polymer. The core includes a TPE and the outer sheath includes a different TPE.

Preferred core components include styrenic-based elastomeric copolymers. Examples include SEBS (Styrene-Ethylene-Butylene-Styrene), available from Shell under the tradename KRATON® , and from Consolidated Polymer Technologies (CPT) under the tradename C-Flex; SEPS (Styrene-Ethylene-Propylene-Styrene), available from M.A. Hanna; and SPBS (Styrene-Propylene-Butylene-Styrene), available from Kuraray Co. The styrenic-based elastomeric block copolymers can be blended with another material, the blend being selected from the group consisting of polyurethane/styrenic-based block copolymer, polyamide/styrenic-based block copolymer, polyester/styrenic-based block copolymer, polyolefin/styrenic-based block copolymer, or maleicanhydride modified styrenic-based block copolymer, and in that the block copolymer is selected from the group consisting of styrene-ethylene-butylene-styrene, styrene-ethylene-propylene-styrene, styrene-propylene-butylene-styrene, styrene-isoprene-styrene, styrene-butadiene-styrene copolymers. Polyether block amides such as those available under the tradename PEBAX® from ELF Atochem may be used if combined with a styrenic-based copolymer, since pure PEBAX cores do not adhere well to gel sheaths. Likewise, (i) polyurethane-based materials (thermoplastic urethanes (TPUs)), such as Tecoflex and Tecothane, both available from Thermedics Inc., Pellathan, available from Dow Chemical, and Elastollan, available from BASF, (ii) polyester-based TPEs, such as Hytrel available from DuPont, (iii) polyolefin-based TPEs, such as SARLINK available from DSM Corp. and SANTOPRENE available from AES Corp., and (iv) caprylactam-based polyurethanes, do not adhere well to gel sheaths when in pure form. Thus, they should also be blended with a styrenic-based copolymer to ensure proper adhesion. Examples of acceptable blended cores are Pellathan/Septon (a styrenic-based copolymer) commercially available from M.A. Hanna as HTE 2203, and a one: one blend of PEBAX MX1205/KRATON FG1901.

Styrenic-based copolymers and blends thereof are also preferred for the sheath material. Preferable sheaths have a Shore A hardness of less than about 10. Preferred TPEs for the sheath that can be used include styrenic-based copolymers such as those available from GLS Corporation (Cary, Illinois) (e.g., LC 115-035B, based on KRATON G-1651); SEPS gel (such as XL0141-x), available from M.A. Hanna, and C-Flex.

The sheath layer may include one or more extenders to increase its softness. Styrenic block copolymers may be extended with a wide variety of materials, as is well known in the art. Suitable extenders include oils, waxes, resins, asphalts, and other polymers, including polyolefins (see, e.g., Technical Bulletin No. SC:1757-93, The Shell Chemical Company describing extenders for KRATON block copolymers). Preferred extenders for increasing the softness of the sheath TPE typically associate with the rubber phase of the copolymer, and include mineral oil (such as Britol, Hydrobrite or Duoprime), silicone fluid, low molecular weight polyethylene, and low molecular weight SEBS/SEPS.

A thermoplastic elastomer and extender are selected to provide a desired combination of softness and resistance of the sheath layer to tearing or abrasion when used. Preferably, the sheath layer material includes a sufficient amount of extender to reduce the Shore A hardness of the material to less than ten.

Various adhesion enhancers such as modifiers to help in processing (e.g. polyolefins), adhesives (e.g., EVA) or tacktifiers (e.g., alpha-methyl vinyl styrene) may be added to either or both of the core and sheath TPEs to enhance the degree of adhesion at the interface therebetween. For example, functionalized (maleic anhydride-grafted) TPE (e.g., KRATON FG1901X, a maleic anhydride-grafted SEBS copolymer) can be added to either one or both of a harder core made of PEBAX MX1205 or Pellathan or Hytrel, and a softer sheath made of KRATON G6713 or other TPE material to improve adhesion. Low molecular weight SEBS can also be added to a TPU for better adhesion. Adhesion can also be improved by adding to the sheath layer a TPE chemically similar to the core material.

Examples of constituted gel materials that may be used with Hanna's HTE 2203 core material are:

| Material | Hanna Gel XL0141-8 (%) | Hanna Gel XL0141-21 (%) | Hanna Gel XL0141-26 (%) |
|---|---|---|---|
| Septon 4055 (SEPS) | 16.8 | 16.8 | 16.6 |
| Britol 50T (High Mol. Wt. Mineral Oil) | 80.0 | -- | -- |
| Irganox 1010 (antiox.) | 0.1 | 0.1 | 0.1 |
| Irgafos 168 (antiox.) | 0.1 | 0.1 | 0.1 |
| Affinity PL 1880 (modifier: low MWPE) | 3.0 | 3.0 | -- |
| Duoprime 90 (mix) | -- | -- | 41.5 |
| Duoprime 90 (inject) | -- | -- | 41.5 |
| Kemamide E (used as an antiox.) | -- | -- | 0.2 |
| Hydrobrite 200 (mix) | -- | 40.0 | -- |
| Hydrobrite 200 (side inject) | -- | 40.0 | -- |

Depending upon the desired characteristics of the floss, it is generally preferred that the floss cross-sectional area has greater than 20% of its area attributed to the strengthening core component, with a preferred ratio or cross-sectional areas of 50% core TPE to 50% sheath TPE. Further, preferred flosses have an outer diameter of from 1.02 to 3.56 mm (0.04 to 0.14 inches). For example, a floss having a HTE 2203 core hardness of 65 Shore A and an SEPS sheath hardness of 0 Shore A should have a diameter of 2.03 mm (0.08). The softer the gel, the wider the floss may be without adversely affecting the user's ability to use the floss. The strength of monofilament floss increases as the core to sheath ratio increases, provided that the core TPE is stronger than the sheath TPE. Whether a gel monofilament is suitable for flossing may be determined by focusing on tear strength and hardness of the monofilament and materials used, rather than solely on tensile strength of the resulting monofilament.

As the hardness of the core material (and therefore its strength and tear resistance) increases, the desirable outer diameter of the floss should decrease, and the ratio between core and sheath should decrease, to ensure proper insertion between a user's teeth. If the outer diameter and ratio remain as large as for softer core materials, then the floss cannot be stretched sufficiently to enable use of the material as a floss. For example, if the core TPE in a core/sheath arrangement has a Shore A hardness of greater than 35, then the outer diameter of the monofilament should be less than 1.78 mm (0.07 inches) and the core/sheath ratio should be 20:80. However, if the core comprises multiple filaments and the sheath material has a hardness of <= 0 Shore A, then the outer diameter can be increased above 1.78 mm (0.07 inches) and can increase the core/sheath ratio above 20:80 to, for example, 50:50.

Typical flosses of the invention have a Shore A hardness of less than 10, an elongation at break of greater than 300%. The floss resulting from combining the core and sheath materials typically can withstand repeated stretching at room temperature to at least twice its original length and will forcibly return to approximately its original length when the tensile force is removed.

TPE components used in the floss of this invention preferably have an elasticity above 200% and preferably above 300%. Preferably, said first elastomer of said core has a viscoelasticity in the range of from 300% to 1200%, and said second elastomer of said sheath has a different viscoelasticity in the range of from 300% to 2000%, more preferably 700% to 2000%.

The tear resistances of the core and sheath materials forming the floss are also important to prevent undue shredding or tearing of the floss during use. Sheath materials preferably have greater than 535 kg/m (30 pounds per linear inch ("pli")), and core materials preferably have greater than 893 kg/m (250 pli), using an ASTM die "C" tear test, ASTM No. D412 run at 23°C. and 51 cm/min. (20 in./min).

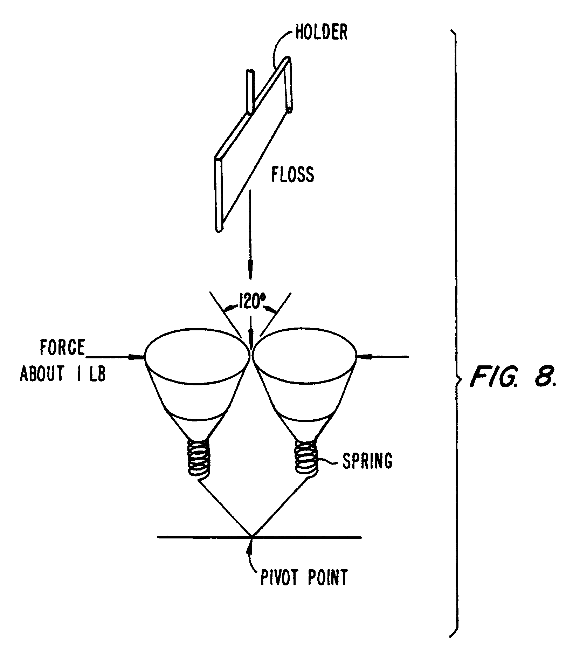

Tear resistance may also be determined by the fray test, which requires stretching the elastic floss to its maximum elongation, mounting the floss on a holder and repeatedly mechanically inserting the stretched floss between a pinch point created by two artificial "teeth". As depicted in Fig. 8, each "tooth" is mounted on a spring to permit a certain amount of give in the apparatus. Both "teeth" are mounted on a single pivot point and are held together by a 0.45 kg (one pound) force. Two sets of teeth were used, a blunt set having a smoothly rounded contact point, and a sharper set having a sharply angled contact points which was used for flosses that could withstand a high number of cycles on the blunt set. The force pushing the teeth together is calibrated by using a piece of nonstretchable material, such as nylon. The normal force measured by the instron to pull a piece of nonstretchable material from the blunt set of teeth was 0.45 kg (1 pound). The number of cycles of insertion before breakage is indicative of the strength and abrasion resistance of the floss. Flosses withstand more than about forty cycles of insertion and retraction between the blunt "teeth" before breaking.

Various combinations of TPEs are shown in the examples. It has been found that best results are achieved if both TPE materials are manageable around the same temperature, and have roughly the same viscosity at handling temperature. If the handling temperature for one TPE differ significantly from that of the other TPE (e.g., greater than 10°C (50°F.)difference), then processing problems can occur in making the multi-component blends. These problems include, for instance, non-even cross-sectional distribution of the core and sheath materials, oddly- or irregularly-shaped product, or ineffective coextrusion because one of the materials is too liquid at the operating temperature required for the other material to maintain its form.

Coextruded monofilament floss has also been produced with a reversed geometry: having the softer gel material form the inner core, surrounded by a thin sheath of the TPE component chosen for strength, elasticity and tear-resistance. For example, the sheath can be made of a PEBAX/nylon blend or polyurethane. These flosses, however, are not as desirable as when the sheath is made of a softer material, because of increased abrasion and less desirable mouth feel.

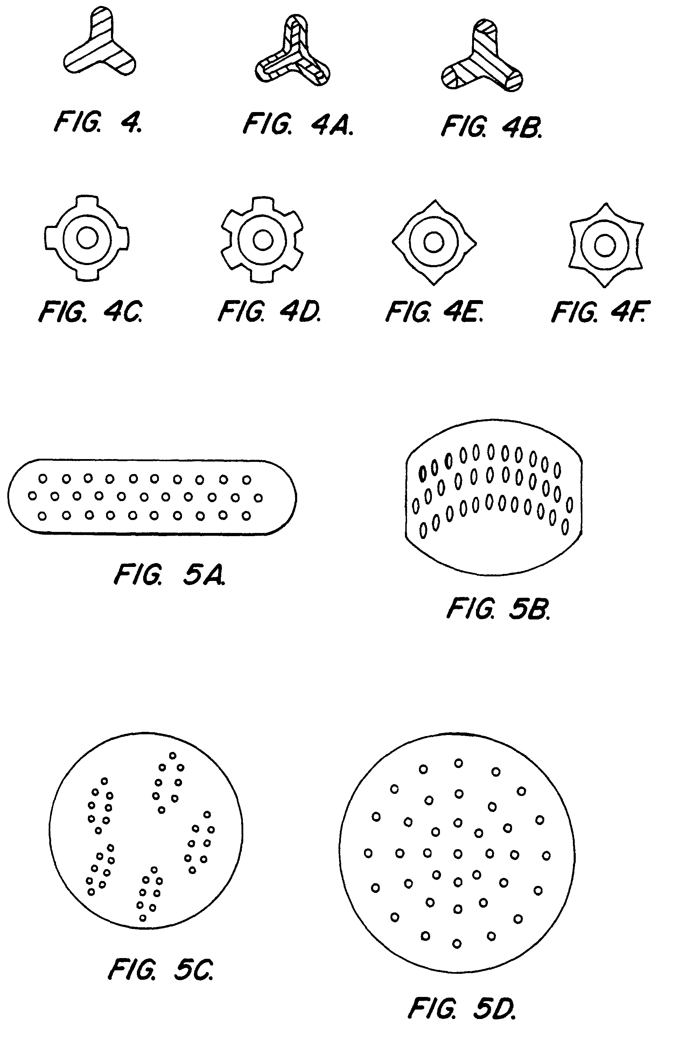

Texturing can be added to the surface of the floss. Texturing improves the feel of floss, and increases user satisfaction with the degree of cleaning achieved by the floss. For example, as shown in Figs. 4c-4f, various geometries of the cross-sectional areas of the monofilaments can be used, including square and triangular bumps. A textured surface increases the user's satisfaction with the floss because of the apparent increased cleaning ability of the floss.

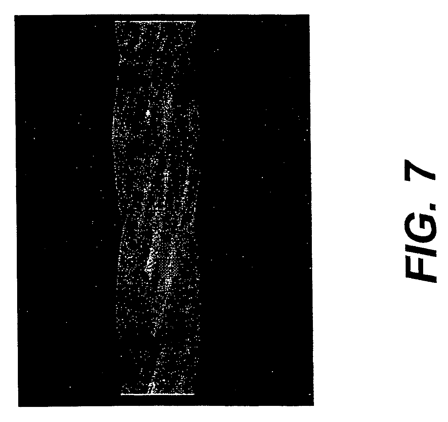

Textured floss can also be created by knitting the core material to produce a bulkier, more irregular surface on which gel is then applied, or, for a multiple filament bundle, by braiding or twisting the multiple filaments before gel-coating the bundle. See, for example, Fig. 7 depicting a twisted monofilament having a cross-section as shown in Fig. 4c.

Another alternative structure for creating a floss having a textured surface is to helically-wrap one or more fine filaments around either the floss core filament or core bundle of filaments, which is then covered with the sheath material, or around the entire floss, so that the wrap material is not concealed from the user's teeth. Since the wrap material (less than 45 denier) is small in diameter when compared to the floss' diameter (for this embodiment, less than 0.51 mm (0.02 inches)), its only purpose being to create an irregular surface, the material must have a higher tensile strength than the core material and must be capable of holding its geometry during use. Nylon has been used successfully for this purpose.

Preferred flosses may also have a "multilobal", "multi-petaled", "multi-spiked", or "multi-spoked" cross-section, as shown in Fig. 4. Preferred filaments include from 3 to 8 "lobes"; one suitable filament has 3 "lobes," as shown in Figs. 4-4b. The filaments are preferably formed by extrusion through a die having the appropriate "multilobal" cross-section. Multicomponent filaments having this cross-sectional configuration may have a sheath/core (Fig. 4a), tipped (Fig. 4b), or other suitable cross-section.

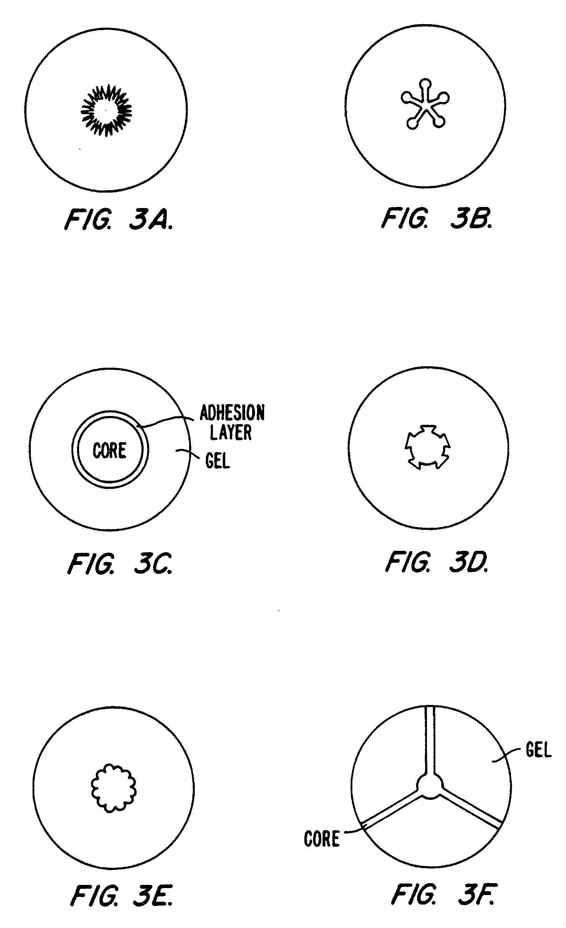

Apart from texturing concerns, the sheath and core may have any suitable cross-section, preferably a symmetric sheath/core cross-section (Fig. 3b) or an eccentric sheath/core cross-section (Fig. 3a). Alternatively, the floss may have a side-by-side arrangement (Fig. 3) or a pie cross-section. (Fig. 3d). The core may have a tubular cross-section along substantially its entire length. Multifilament arrangements are also possible, such as depicted in Figs. 5a-5d ("islands-in-the-sea", and figures depicting the "islands-in-the-sea" and coated multifilament embodiments as disclosed in the U.S. Patent Application entitled " 'Satin' Dental Floss," filed concurrently herewith. These arrangements permit increased adhesion between the core and sheath materials by increasing the surface area across which the materials are in contact.

Adhesion can also be improved between core and sheath materials either by varying the geometry of a single core to increase the surface area of contact between the core and sheath materials, as disclosed in Figs. 3a, 3e and 3f, and by creating mechanical interlocking structures between the core and sheath materials, as disclosed in Figs. 3b and 3d. A separate adhesive layer may also be included between the gel sheath and core to ensure adhesion between the two. Inclusion of such a layer, however, increases the complexity of manufacturing such a floss.

### Additives

The described preferred embodiments can be formulated to include one or more additives, e.g., a color, flavor, or active ingredient, in one or more of the components of the floss. For example, the sheath layer may contain an abrasive for improved cleaning such as kaolin, clay, and silica, or flavors and pigments to flavor and color the floss. Examples of such additives are chlorhexidine (or a salt thereof), sodium fluoride, flavor (e.g. Polyiff® , or peppermint oil, from International Flavors and Fragrances Co. or Quest International Fragrance Inc.), fragrance, tooth desensitizer, tooth whitener, pigments, e.g., titanium dioxide, to impart color to the floss, or antioxidants, to prevent discoloration, or other additives suitable for use in dental flosses such as antimicrobial agents. The preferable TPE for carrying the additive will be determined by the additive used, as would be readily appreciated by one skilled in the art.

The additive-containing component(s) may be water-soluble, to allow the additive to leach from the floss during use. The additive may also be provided as supplied, in microencapsulated form, or adsorbed or absorbed onto another additive, e.g., a particulate filler. Flavoring, for example, can also be released through a floss by incorporating the fluid into a floss with a tubular core.

The additive, if desired, can be incorporated in encapsulated form. Encapsulation may be used for thermal protection or moisture protection of the additive, and may be accomplished by any number of conventional techniques, such as spray drying spray-chilling, drum drying or solvent evaporation.

Advantageously, additives in liquid and/or encapsulated form can be incorporated into the flosses of the invention during manufacture of the filaments, rather than applying the additives later during separate coating steps. This not only reduces the number of processing steps, but also reduces the amount of additive needed.

How flavor oils, for example, are added to the floss depends on whether the materials in the floss are susceptible to breakdown by the oils, all as known in the art. Some materials, such as polyether polyurethanes and KRATON, are not attacked by flavor oils. Liquid flavors can therefore be dip-coated or sprayed directly onto the floss itself without concern about the floss' integrity, all as appreciated by one skilled in the art.

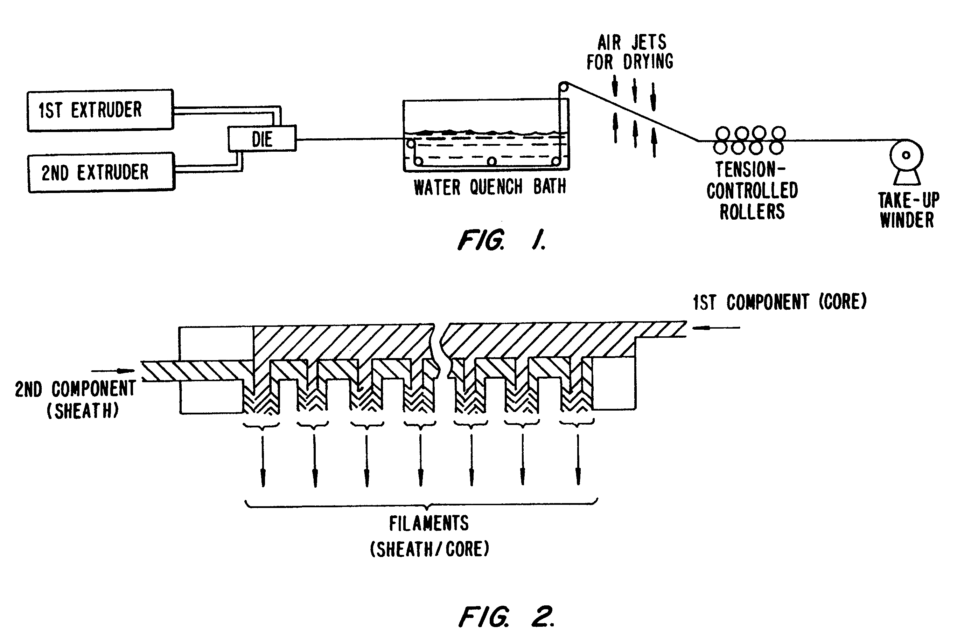

A preferred method for forming a dental floss of the invention is shown schematically in Fig. 1. First, two or more TPEs are coextruded through a two-component extrusion die. The polymers are chosen to produce a floss having the desired physical properties and/or the relative cross-sections, as described above. Preferred outer diameters of the holes in the die are from 1.02 to 3.56 mm (0.04 to 0.14 inches). The die block is fitted with heater probes and a thermocouple. The heater is set at a temperature sufficient to avoid solidifying both TPEs. Suitable temperatures for the die block to permit the TPEs to easily flow can be determined empirically for a particular elastomer or elastomer/extender blend.

The filament exiting the die then passes through a water quenching chamber. The orientation of the die will dictate the location of this chamber. If the coextrudate exits the die horizontally (as depicted), then the water quenching chamber will be located directly downstream as close as possible to the spinneret, to minimize the opportunity for the horizontally-disposed filament to deform before quenching due to gravity. If the coextrudate is produced in a vertical stream, however, then deformation of the molten polymer stream due to gravity is not as great a concern, and the water bath may be placed further away, although still preferably less than two feet from the die. The monofilament fiber is guided under low tension from the extruder through the quench chamber and, upon exiting the bath, is passed over a series of tension-controlled rollers before being wound-up under low tension. The speed of the tension rollers controls the size of the final filaments. Preferred speeds of travel of the fiber through the above process are from roughly 45.7 m (150 feet) per minute for commercial processing. No draw-down is necessary for these filaments.

Twisted flosses as shown in Fig. 7 may be produced by twisting the hot molten extrudate before setting in the cold water bath. Alternatively, post-coextrusion treatment of the floss by heating to approximately 121°C. (250°F.), twisting, and then setting in cold water will produce the same result.

Crimped or embossed floss may be produced by passing the hot extrudate, before quenching in the water bath, through an opposing set of chilled and motorized aluminum wheels containing a textured groove. The depth of the groove is roughly half that of the extrudate, so that grooves in two opposed wheels permit the floss to pass through and be textured without substantial deformation. Patterns etched into the grooves transfer to the extrudate as it passes through the grooves while being quenched. Patterns may take any form although cross-hatched or screw-type impressions are preferred. The speed of the wheels should be set to match the extrusion speed. The wheels should be coated with teflon, or wet with water to prevent the hot extrudate from sticking to the wheels.

Alternatively, if the floss is to be crimped after the floss has already been cooled, then a heated (up to around 37.8°C. (100°F.)) grooved wheel may be used, after which the floss is requenched. The small amount of heat is sufficient to soften the outer layer of the floss to permit crimping.

If floss is braided or knitted, soft sheath material can be coated in a layer thin enough so that the outer surface of the floss has a geometry corresponding to the outer surface of the braided or knitted portion.

In another embodiment of the invention, multiple filaments of one or more components are extruded as taught in the U.S. Patent Application entitled " 'Satin' Dental Floss," filed concurrently herewith. For example, multiple filaments of Elastollan are extruded, water-quenched, and heat drawn in a 2:1 ratio to orient the individual filaments. SEPS gel is then pressure-coated, or "pulltruded," onto the surface of the horizontally-oriented bundle by drawing the filaments through a single-hole die to which the SEPS gel is applied. Preferred bundles of multiple filaments have 144, 96 or 48 filaments.

Finally, floss may be packaged in any manner. One preferred manner is to package approximately fifteen cm (six-inch) relaxed lengths of the floss in a gas-impermeable material to maintain sterility and prevent excessive evaporation of the flavor oils. The package is attached at either end of the floss, so that when the packaging is torn, the package ends provide useful tabs for the user to hold while flossing. Another manner is to package the floss under low-tension on a small bobbin.

### Examples

The following examples are put forth so as to provide those of ordinary skill in the art with a complete disclosure and description of how to make the monofilament flosses and carry out the extrusion methodology of the invention, and are not intended to limit the scope of what the inventors regard as their invention. Efforts have been made to ensure accuracy with respect to numbers used (e.g., amounts, temperatures, etc.) but some experimental errors in deviation should be accounted for.

The following description of an equipment set-up and manufacturing procedure is representative of that used to create the flosses described. Two 3.18 cm (1.25-inch) diameter extruders were connected to a two-component extrusion die with metering pumps on each screw operating to deliver the flowrate of material to the die to form a floss having a ratio of 50:50 core material:gel material. The two-component extrusion die included a metering plate, a distributing plate, etched plates, and a spinneret/ die. Operating temperatures of the equipment and molten materials were recorded at various stages before leaving the die. After being coextruded through the extrusion die according to standard methods, the extrudate was processed with a downstream filament spinning set-up to produce floss. The downstream set-up included a quenching water bath, tension-controlled rollers and a winder.

Using the equipment set-up and procedures described above, the following specialty bicomponent, monofilament flosses were formed, which exhibited improved mechanical properties over a single component "gel" monofilament:

| Example No.: | 1 | 2 |
|---|---|---|
| Date of Run | 06/17/96 | 03/07/96 |
| Cross-section | 34 islands/sea rod; Fig. 6b | 34 islands/sea tape |
| Core Component | TPU/KRAT. HTE 2203 | TPU/KRAT. HTE 2203 |
| Sheath Comp. | SEPS gel XL0141-8 | GLS G6713 KRATON |
| Ratio (Sheath/Core) | 50/50 | 50/50 |
| Sheath O.D./ Core O.D. cm (in.) | Rod 0.20 cm (0.08") O.D. | 0.0165 cm (0.0065") x 0.945 cm (0.372") |
| Metering Pumps (size (cc)/speed (rpm)) | 6 cc / 5 rpm both | 6 cc / 3 rpm both |
| Temp. of Core mat'l at die exit (°C.) | 201 | 199 |
| Temp. of Sheath mat'l at die exit (°C.) | 205 | 200 |
| Temp. of Spin Head (°C.) | 210 | 203 |
| Winder Speed (mpm) | 20 | Manual wind |
| Fray Test (avg. of 5 runs) | 147+/- 8 Blunt | 197 Blunt; 3.2+/- 0.2 Sharp |
| Tensile strength (kg) | 3.1 +/- 0.1 | not measured |
| Comments | -- | 15 mm x 0.17 mm slot die; vertical extrusion |

| Example No.: | 3 | 4 |
|---|---|---|
| Date of Run | 02/14/96 | 01/26/96 |
| Cross-section | Core/sheath | Core/sheath tube; Fig. 4c |
| Core Component | Hanna HTE 1113 (Shore A 66 SBS) | PEBAX MX1205 / KRATON FG1901 1:1 blend |
| Sheath Comp. | GLS LC115-035B | GLS LC 115-035B |
| Ratio (Sheath/Core) | 80/20 | 80/20 |
| Sheath O.D./ Core O.D. cm (in.) | 0.18 cm / 0.081 cm (0.07" / 0.032") | 0.152 cm / 0.081 cm (0.060" / 0.032"') tube 0.051 cm (0.020") |
| Metering Pumps (size / speed (rpm)) | 1 amp/19.5 rpm for sheath mat'l; no melt pump for core mat'l | 1 amp/19.5 rpm for sheath mat'l; no melt pump for core mat'l |
| Temp. of Core mat'l at die exit (°C.) | not measured | not measured |
| Temp. of Sheath mat'l at die exit (°C.) | not measured | not measured |
| Temp. of Spin Head (°C.) | 182 | 182 |
| Winder Speed (mpm) | 6.79 m/min (22.3 ft/min.) | approx 6.7 m/min (22 ft./min.) |
| Fray Test (avg. of 5 runs) | 24 +/-1 Blunt | 62 +/- 10 Blunt |

| Example No.: | 5 |
|---|---|
| Date of Run | 03/07/96 |
| Cross-section | Multiple Bicomponent Filaments |
| Core Component | Hanna TPU/KRATON blend HTE 2203 |
| Sheath Comp. | GLS KRATON G6713 |
| Ratio (Sheath/Core) | 30/70 |
| Sheath O.D. / Core O.D. (in.) | -- |
| Metering Pumps (size (cc) / speed (rpm)) | 6cc / 2.4 rpm (sheath) 6cc / 5.6 rpm (core) |
| Temp. of Core mat'l at die exit (°C.) | 196 |
| Temp. of Sheath mat'l at die exit (°C.) | 197 |
| Temp. of Spin Head (°C.) | 203 |
| Winder Speed | hand-wound |
| Fray Test (avg. of 5 runs) | 96 filaments: 32+/-2 cycles (Sharp) 48 filaments: 37+/-3 cycles (Sharp) |

Other embodiments are within the claims. For example, while bicomponent monofilaments have been described above in the Detailed Description; the filaments could contain any desired number of components, and in this case would be manufactured by extrusion through a suitable multicomponent die using the appropriate number of extruders.

## Claims

1. A multicomponent, monofilament, viscoelastic dental floss **characterized by** a core composed of a first thermoplastic elastometer and a sheath encasing said core composed of a second, different thermoplastic elastometer, the difference of the melting points of said first and second thermoplastic elastomers being less than 30°C., said floss having a Shore A hardness of less than 10 and an ultimate elongation of greater than 300, said floss having a tear resistance of greater than 40 cycles on a blunt-toothed fray test.

2. Dental floss according to claim 1, **characterized in that** at least one of the core and sheath thermoplastic elastomers comprises a styrenic-based block copolymer.

3. Dental floss according to claim 2, **characterized in that** said block copolymer is blended with another material, said blend improving the chemical affinity between the core and sheath elastomers and being selected from the group consisting of polyurethane/styrenic-based block copolymer, polyamide/styrenic-based block copolymer, polyester/styrenic-based block copolymer, polyolefin/styrenic-based block copolymer, or maleicanhydride modified styrenic-based block copolymer, and **in that** the block copolymer is selected from the group consisting of styrene-ethylene-butylene-styrene, styrene-ethylene-propylene-styrene, styrene-propylene-butylene-styrene, styrene-isoprene-styrene, styrene-butadiene-styrene copolymers.

4. Dental floss according to claim 1, **characterized in that** the sheath further comprises an extender to increase the softness of said sheath and being selected from the group consisting of oils, waxes, resins and asphalts.

5. Dental floss according to claim 1, **characterized in that** one or more of the elastomers of the floss includes an additive selected from the group consisting of colors, fragrances, flavors, therapeutically-active ingredients, and modifiers which improve the adhesion between the elastomers of the core and sheath.

6. Dental floss according to claim 5, **characterized in that** said modifier is selected from the group consisting of functionalized thermoplastic elastomers, thermoplastic elastomers having the same block copolymer structure as the thermoplastic elastomer in the corresponding portion of said floss, tacktifiers, or adhesives.

7. Dental floss according to claim 1, **characterized in that** said first elastomer of said core has a viscoelasticity in the range of from 300% to 1200%, and said second elastomer of said sheath has a different viscoelasticity in the range of from 300% to 2000%.

8. Dental floss according to claim 1, **characterized in that** said core has geometrically-enlarged surface area to increase adhesion between the core and the sheath.

9. Dental floss according to claim 8, **characterized in that** said core has a cross-sectional shape that is multi-lobed, multi-petaled, multi-spiked or multi-spoked.

10. Dental floss according to claim 1, **characterized in that** said core has geometrically-enlarged surface area that comprises means for interlocking the core and sheath for enhancing adhesion therebetween.

11. Dental floss according to claim 1, **characterized in that** said floss has a cylindrical cross-section with an outer diameter of from 1.02 to 3.56 mm (0.04 inches to 0.14 inches).

12. Dental floss according to claim 1, **characterized in that** said floss has a textured outer surface.

13. Dental floss according to claim 1, **characterized in that** said core has a tubular cross-section along substantially its entire length.

14. A multicomponent, monofilament, viscoelastic dental floss **characterized by** a plurality of continuous cores having a first thermoplastic elastomer and a body sheath substantially adhered to and substantially surrounding each of said cores along substantially the entire lengths thereof comprising a second thermoplastic elastomer, the difference of the melting points of said first and second thermoplastic elastomers being less than 10°C. (50°F.), said floss having a Shore A hardness of less than 10 and an ultimate elongation of greater than 300, said floss having a tear resistance of greater than 40 cycles on a blunt-toothed fray test.

15. Dental floss according to claim 14, **characterized in that** said plurality of continuous cores are arranged in multiple groupings of cores spaced throughout the floss' cross-sectional area.

16. A method of making a multicomponent, monofilament, viscoelastic dental floss of claim 1, **characterized by** the steps of coextruding two or more thermoplastic elastomers through a multicomponent die assembly to form extrudate comprising said at least one core having said first thermoplastic elastomer and said sheath comprising said second thermoplastic elastomer, and quenching said coextrudate.

17. A method of making a multicomponent, monofilament, viscoelastic dental floss of claim 14, **characterized by** the steps of coextruding two or more materials through a multicomponent die assembly to form coextrudate comprising said plurality of cores having said first thermoplastic elastomer embedded in said sheath comprising said second thermoplastic elastomer, and quenching said coextrudate.

## Patentansprüche

1. Monofile, viskoelastische Mehrkomponenten-Zahnseide, **gekennzeichnet durch** einen Kern aus einem ersten thermoplastischen Elastomer und einen den Kern umschließenden Mantel aus einem davon verschiedenen zweiten thermoplastischen Elastomer, wobei der Unterschied in den Schmelzpunkten des ersten und zweiten thermoplastischen Elastomers weniger als 30°C beträgt, wobei die Zahnseide eine Härte Shore A von weniger als 10 und eine Reißdehnung von mehr als 300 aufweist, und wobei die Zahnseide bei einem Zerfaserungstest mit stumpfen Zähne eine Reißfestigkeit von mehr als 40 Zyklen hat.

2. Zahnseide nach Anspruch 1, **dadurch gekennzeichnet, daß** wenigstens eines der thermoplastischen Elastomere von Kern und Mantel ein styrolhaltiges Blockcopolymer umfaßt.

3. Zahnseide nach Anspruch 2, **dadurch gekennzeichnet, daß** das Blockcopolymer mit einem weiteren Material vermischt ist, wobei die Mischung die chemische Affinität zwischen den Elastomeren von Kern und Mantel verbessert und ausgewählt ist aus der aus einem polyurethan-/styrolhaltigen Blockcopolymer, einem polyamid-/styrolhaltigen Blockcopolymer, einem polyester-/styrolhaltigen Blockcopolymer, einem polyolefin/styrolhaltigen Blockcopolymer, oder einem mit Maleinsäureanhydrid modifizierten styrolhaltigen Blockcopolymer bestehenden Gruppe, und daß das Blockcopolymer ausgewählt ist aus der aus Styrol-Ethylen-Butylen-Styrol-, Styrol-Ethylen-Propylen-Styrol-, Styrol-Propylen-Butylen-Styrol-, Styrol-Isopren-Styrol- und Styrol-Butadien-Styrol-Copolymeren bestehenden Gruppe.

4. Zahnseide nach Anspruch 1, **dadurch gekennzeichnet, daß** der Mantel ferner ein Streckmittel umfaßt, um die Weichheit des Mantels zu erhöhen, wobei das Streckmittel ausgewählt ist aus der aus Ölen, Wachsen, Harzen und Asphalten bestehenden Gruppe.

5. Zahnseide nach Anspruch 1, **dadurch gekennzeichnet, daß** mindestens eines der Elastomere der Zahnseide einen Zusatz umfaßt, der ausgewählt ist aus der aus Farben, Duftstoffen, Geschmacksstoffen, therapeutisch wirksamen Bestandteilen und Modifikatoren bestehenden Gruppe, wobei diese Stoffe die Haftung zwischen den Elastomeren von Kern und Mantel erhöhen.

6. Zahnseide nach Anspruch 5, **dadurch gekennzeichnet, daß** der Modifikator ausgewählt ist aus der aus funktionalisierten thermoplastischen Elastomeren, thermoplastischen Elastomeren mit derselben Blockcopolymerstruktur wie das thermoplastische Elastomer in dem entsprechenden Abschnitt der Zahnseide, Klebrigmachern bzw. Klebstoffen bestehenden Gruppe.

7. Zahnseide nach Anspruch 1, **dadurch gekennzeichnet, daß** das erste Elastomer des Kerns eine Viskoelastizität im Bereich von 300 % bis 1200 % hat und das zweite Elastomer des Mantels eine davon verschiedene Viskoelastizität im Bereich von 300 % bis 2000 % hat.

8. Zahnseide nach Anspruch 1, **dadurch gekennzeichnet, daß** der Kern eine geometrisch vergrößerte Oberfläche hat, um die Haftung zwischen Kern und Mantel zu erhöhen.

9. Zahnseide nach Anspruch 8, **dadurch gekennzeichnet, daß** der Kern eine Querschnittsform hat, die viellappig, vielblättrig, vielzackig oder vielspeichig ist.

10. Zahnseide nach Anspruch 1, **dadurch gekennzeichnet, daß** der Kern eine geometrisch vergrößerte Oberfläche hat, die eine Einrichtung zum Verzahnen von Kern und Mantel aufweist, um die Haftung dazwischen zu verbessern.

11. Zahnseide nach Anspruch 1, **dadurch gekennzeichnet, daß** die Zahnseide einen zylindrischen Querschnitt hat mit einem Außendurchmesser von 1,02 bis 3,56 mm (0,04 Inch bis 0,14 Inch).

12. Zahnseide nach Anspruch 1, **dadurch gekennzeichnet, daß** die Zahnseide eine texturierte Außenseite hat.

13. Zahnseide nach Anspruch 1, **dadurch gekennzeichnet, daß** der Kern im wesentlichen entlang seiner gesamten Länge einen rohrförmigen Querschnitt hat.

14. Monofile, viskoelastische Mehrkomponenten-Zahnseide, **gekennzeichnet durch** mehrere zusammenhängende Kerne aus einem ersten thermoplastischen Elastomer und **durch** einen Mantel aus einem zweiten thermoplastischen Elastomer, der im wesentlichen entlang der gesamten Länge der Kerne im wesentlichen an jedem der Kerne haftet und jeden dieser Kerne im wesentlichen umschließt, wobei der Unterschied in den Schmelzpunkten des ersten und zweiten thermoplastischen Elastomers weniger als 10°C (50°F) beträgt, wobei die Zahnseide eine Härte Shore A von weniger als 10 und eine Reißdehnung von mehr als 300 hat, und wobei die Zahnseide bei einem Zerfaserungstest mit stumpfen Zähnen eine Reißfestigkeit von mehr als 40 Zyklen hat.

15. Zahnseide nach Anspruch 14, **dadurch gekennzeichnet, daß** die mehreren zusammenhängenden Kerne über die gesamte Querschnittsfläche der Zahnseide in einer Vielzahl von Gruppen von Kernen angeordnet sind.

16. Verfahren zur Herstellung einer monofilen, viskoelastischen Mehrkomponenten-Zahnseide nach Anspruch 1, **gekennzeichnet durch** die folgenden Schritte: Zwei oder mehr thermoplastische Elastomere werden **durch** eine Mehrkomponentendüse koextrudiert, um ein Extrudat zu bilden, das aus dem aus dem ersten thermoplastischen Elastomer bestehenden wenigstens einen Kern und aus dem aus dem zweiten thermoplastischen Elastomer bestehenden Mantel besteht, und das Koextrudat wird abgeschreckt.

17. Verfahren zur Herstellung einer monofilen, viskoelastischen Mehrkomponenten-Zahnseide nach Anspruch 14, **gekennzeichnet durch** die folgenden Schritte: Zwei oder mehr Materialien werden **durch** eine Mehrkomponentendüse koextrudiert, um ein Koextrudat zu bilden, das aus den aus dem ersten thermoplastischen Elastomer bestehenden mehreren Kernen besteht, die in den aus dem zweiten thermoplastischen Elastomer bestehenden Mantel eingebettet sind, und das Koextrudat wird abgeschreckt.

## Revendications

1. Fil dentaire multiconstituant, à monofilament, viscoélastique **caractérisé par** un noyau constitué d'un premier élastomère thermoplastique et d'une enveloppe enfermant ledit noyau constituée d'un second élastomère thermoplastique différent, la différence des points de fusion desdits premier et second élastomères thermoplastiques étant inférieure à 30°C, ledit fil ayant une dureté Shore A inférieure à 10 et un allongement final supérieur à 300, ledit fil ayant une résistance à la déchirure supérieure à 40 cycles sur un test d'effilochage sur dents émoussées.

2. Fil dentaire selon la revendication 1, **caractérisé en ce qu'**au moins un des élastomères thermoplastiques de noyau et d'enveloppe comprend un copolymère séquencé à base de styrène.

3. Fil dentaire selon la revendication 2, **caractérisé en ce que** ledit copolymère séquencé est mélangé avec un autre matériau, ledit mélange améliorant l'affinité chimique entre les élastomères de noyau et d'enveloppe et étant choisi parmi un copolymère séquencé à base de polyuréthane/styrène, un copolymère séquencé à base de polyamide/styrène, un copolymère séquencé à base de polyester/styrène, un copolymère séquencé à base de polyoléfine/styrène ou un copolymère séquencé à base de styrène modifié par de l'anhydride maléique et **en ce que** le copolymère séquencé est choisi parmi des copolymères de styrène-éthylène-butylène-styrène, de styrène-éthylène-propylène-styrène, de styrène-propylène-butylène-styrène, de styrène-isoprène-styrène, de styrène-butadiène-styrène.

4. Fil dentaire selon la revendication 1, **caractérisé en ce que** l'enveloppe comprend en outre un diluant pour augmenter la souplesse de ladite enveloppe et choisi parmi des huiles, des cires, des résines et des asphaltes.

5. Fil dentaire selon la revendication 1, **caractérisé en ce qu'**un ou plusieurs des élastomères du fil comprennent un additif choisi parmi des couleurs, des parfums, des arômes, des ingrédients thérapeutiquement actifs et des agents de modification qui améliorent l'adhérence entre les élastomères du noyau et de l'enveloppe.

6. Fil dentaire selon la revendication 5, **caractérisé en ce que** ledit agent de modification est choisi parmi des élastomères thermoplastiques rendus fonctionnels, des élastomères thermoplastiques ayant la même structure de copolymère séquencé que l'élastomère thermoplastique dans la partie correspondante dudit fil, des agents collants ou des adhésifs.

7. Fil dentaire selon la revendication 1, **caractérisé en ce que** ledit premier élastomère dudit noyau présente une viscoélasticité dans l'intervalle de 300 % à 1200 % et ledit second élastomère de ladite enveloppe présente une viscoélasticité différente dans l'intervalle de 300 % à 2000 %.

8. Fil dentaire selon la revendication 1, **caractérisé en ce que** ledit noyau présente une surface spécifique géométriquement agrandie pour augmenter l'adhérence entre le noyau et l'enveloppe.

9. Fil dentaire selon la revendication 8, **caractérisé en ce que** ledit noyau présente une forme transversale qui est à lobes multiples, à pétales multiples, à épis multiples ou à pointes multiples.

10. Fil dentaire selon la revendication 1, **caractérisé en ce que** ledit noyau présente une surface spécifique géométriquement agrandie qui comprend un moyen pour enchevêtrer le noyau et l'enveloppe pour promouvoir l'adhérence entre ceux-ci.

11. Fil dentaire selon la revendication 1, **caractérisé en ce que** ledit fil présente une section transversale cylindrique avec un diamètre externe de 1,02 à 3,56 mm (0,04 pouce à 0,14 pouce).

12. Fil dentaire selon la revendication 1, **caractérisé en ce que** ledit fil présente une surface externe texturée.

13. Fil dentaire selon la revendication 1, **caractérisé en ce que** ledit noyau présente une section transversale tubulaire le long de sa longueur pratiquement totale.

14. Fil dentaire multiconstituant, à monofilament, viscoélastique **caractérisé par** de nombreux noyaux continus ayant un premier élastomère thermoplastique et une enveloppe de corps collée pratiquement à et entourant pratiquement chacun desdits noyaux le long des longueurs pratiquement totales de ceux-ci comprenant un second élastomère thermoplastique, la différence des points de fusion desdits premier et second élastomères thermoplastiques étant inférieure à 10°C (50°F), ledit fil ayant une dureté Shore A inférieure à 10 et un allongement final supérieur à 300, ledit fil ayant une résistance au déchirement supérieure à 40 cycles sur un test d'effilochage sur dents émoussées.

15. Fil dentaire selon la revendication 14, **caractérisé en ce que** lesdits nombreux noyaux continus sont disposés dans des groupements multiples de noyaux espacés d'un bout à l'autre de la surface transversale du fil.

16. Procédé de fabrication d'un fil dentaire multiconstituant, à monofilament, viscoélastique selon la revendication 1, **caractérisé par** les étapes de coextrusion de deux ou plusieurs élastomères thermoplastiques par l'intermédiaire d'un assemblage de filière à constituants multiples pour former un produit d'extrusion comprenant ledit au moins un noyau ayant ledit premier élastomère thermoplastique et ladite enveloppe comprenant ledit second élastomère et de trempe dudit produit de coextrusion.

17. Procédé de fabrication d'un fil dentaire multiconstituant, à monofilament, viscoélastique selon la revendication 14, **caractérisé par** les étapes de coextrusion de deux ou plusieurs matières à travers un assemblage de filière à constituants multiples pour former un produit de coextrusion comprenant lesdits nombreux noyaux ayant ledit premier élastomère thermoplastique encastré dans ladite enveloppe comprenant ledit second élastomère thermoplastique et de trempe dudit produit de coextrusion.
